# EUROPEAN PATENT SPECIFICATION

(11) **EP 2 473 046 B1**
(45) Date of publication and mention of the grant of the patent: **22.10.2014**
(21) Application number: 10814412.2
(22) Date of filing: 01.09.2010
(51) Int. Cl.: A01N 43/40, A61K 31/445, A61P 31/12

(54) **IMINOSUGARS FOR THEIR USE IN THE TREATMENT OF FILOVIRAL DISEASES**
IMINOZUCKER ZUR VERWENDUNG IN DER BEHANDLUNG VON FILOVIRUSERKRANKUNGEN
IMINOSUCRES POUR LEUR UTILISATION DANS LE TRAITEMENT DES MALADIES FILOVIRALES

(30) Priority: 22.02.2010 US 282507 P; 04.09.2009 US 272253 P
(43) Date of publication of application: 11.07.2012
(73) Proprietor: United Therapeutics Corporation, Silver Spring, MD 20910 (US); THE CHANCELLOR, MASTERS AND SCHOLARS OF THE UNIVERSITY OF OXFORD, Oxford OX1 2JD (GB)
(72) Inventor: RAMSTEDT, Urban, Silver Spring, MD 20910 (US); KLOSE, Brennan, Silver Spring, MD 20910 (US); ZITZMANN, Nicole, Oxford OX1 4TL (GB); DWEK, Raymond, A., Oxford OX2 9AU (GB); BUTTERS, Terry, D., Oxford OX44 9BS (GB)
(74) Representative: HOFFMANN EITLE
(86) International application number: PCT/US2010/047493
(87) International publication number: WO 2011/028779

(56) References cited:
- WO-A1-2010/027996
- WO-A2-2006/077427
- WO-A2-2007/140184
- WO-A2-2010/109330
- US-A- 4 182 767
- US-A1- 2006 074 107
- US-A1- 2006 264 467
- US-A1- 2009 186 847
- MEHTA ANAND ET AL: "Imino sugars that are less toxic but more potent as antivirals, in vitro, compared with N-n-nonyl DNJ", ANTIVIRAL CHEMISTRY & CHEMOTHERAPY, BLACKWELL SCIENTIFIC PUBL., LONDON, GB, vol. 13, no. 5, 1 September 2002 (2002-09-01), pages 299-304, XP009154285, ISSN: 0956-3202
- SILBER AM, CANDURRA NA, DAMONTE EB.: "The effects of oligosaccharide trimming inhibitors on glycoprotein expression and infectivity of Junin virus.", FEMS MICROBIOL LETT., vol. 109, no. 1, 1 May 1993 (1993-05-01), pages 39-43,
- HUANG R, ET AL: 'Antiviral activity of some natural and synthetic sugar analogues.' FEBS LETT. vol. 291, no. 2, 21 October 1991, pages 199 - 202
- SUNKARA P.S ET AL: 'Antiretroviral activity of castanospermine and deoxynojirimycin, specific inhibitors of glycoprotein processing.' BIOCHEM BIOPHYS RES COMMUN. vol. 148, no. 1, 14 October 1987, pages 206 - 210
- WRIGHT K.E. ET AL: 'Post-translational processing of the glycoproteins of lymphocytic choriomeningitis virus.' VIROLOGY vol. 177, no. 1, 01 July 1990, pages 175 - 183

## Description

### FIELD

The present application relates to iminosugars for use in a method of treating viral infections and, in particular, to iminosugars for use in methods of treatment and prevention of viral infections caused by a virus belonging to the Filoviridae family.

### SUMMARY

One embodiment is a compound of the formula, 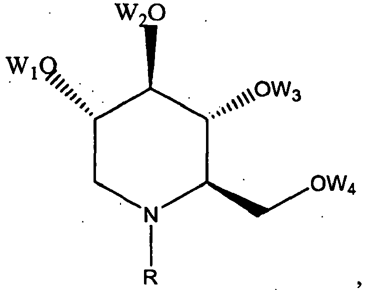 or a pharmaceutically acceptable salt thereof, for use in a method of treating or preventing a disease or condition caused by a virus belonging to the Filoviridae family,
wherein R is either selected from substituted or unsubstituted alkyl groups, substituted or unsubstituted cycloalkyl groups, substituted or unsubstituted aryl groups, or substituted or unsubstituted oxaalkyl groups; or wherein R is 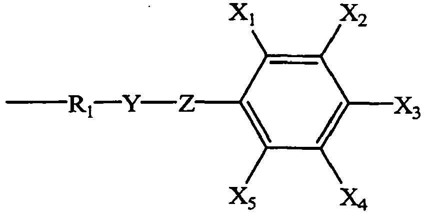 R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl; and
wherein W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

WO 2006/077427 discloses a combined preparation comprising a glycosylation modulator and a membrane fusion inhibitor for combined, simultaneous or sequential use in the treatment of infections caused by viruses bearing glycosylated envelope proteins.

### DRAWINGS

Figures 1(A)-(E) present chemical formulas of the following iminosugars: A) *N*-Butyl deoxynojirimycin (NB-DNJ or UV-1); B) *N*-Nonyl deoxynojirimycin (NN-DNJ or UV-2); C) *N*-(7-Oxadecyl)deoxynojirimycin (N7-O-DNJ or UV-3); D) *N*-(9-Methoxynonyl) deoxynojirimycin (N9-DNJ or UV-4); E) *N*-(*N*-{4'-azido-2'-nitrophenyl}-6-aminohexyl)deoxynojirimycin (NAP-DNJ or UV-5).

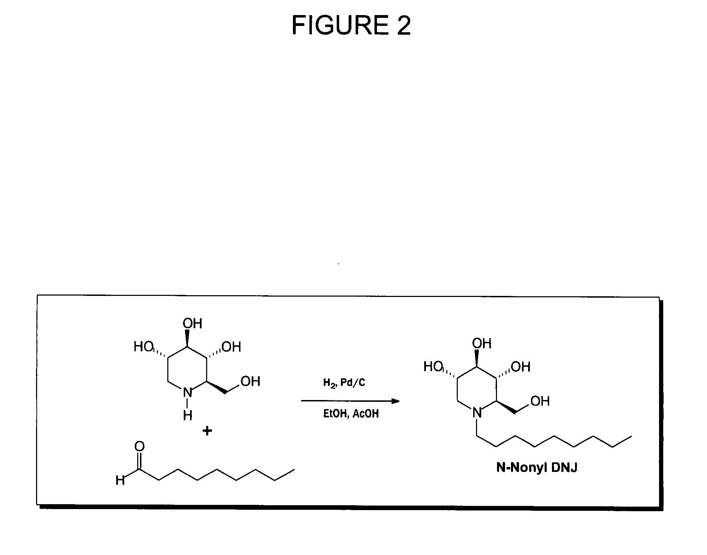

Figure 2 is a synthesis scheme for NN-DNJ.

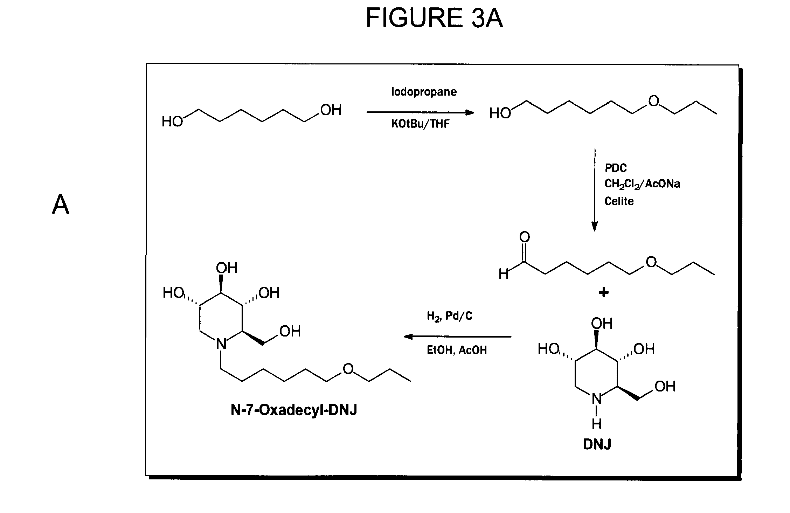

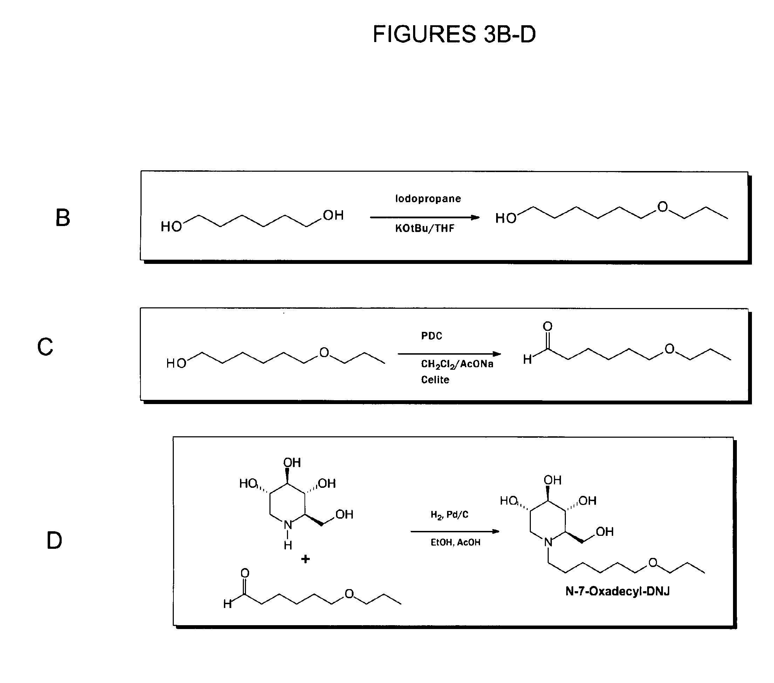

Figures 3A-D illustrate synthesis of N7-Q-DNJ. In particular, Figure 3A shows a sequence of reactions leading to N7-O-DNJ; Figure 3B illustrates preparation of 6-propyloxy-1-hexanol; Figure 3C illustrates preparation of 6-propyloxy-1-hexanal; Figure 3D illustrates synthesis of N7-O-DNJ.
Figures 4A-C relate to synthesis of *N*-(9-Methoxynonyl) deoxynojirimycin. In particular,

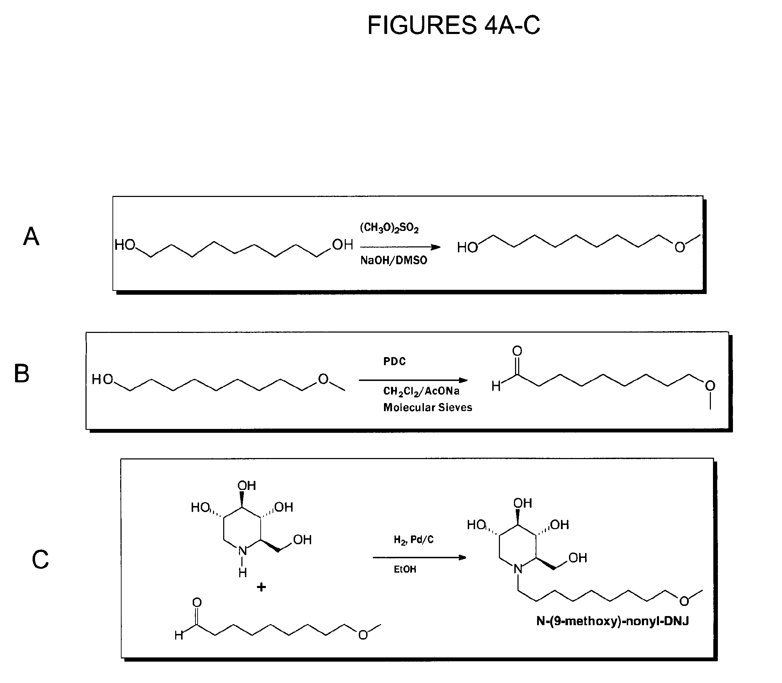

Figure 4A illustrates preparation of 9-methoxy-1-nonanol; Figure 4B illustrates preparation of 9-methoxy-1-nonanal; Figure 4C illustrates synthesis of *N*-(9-Methoxynonyl) deoxynojirimycin.

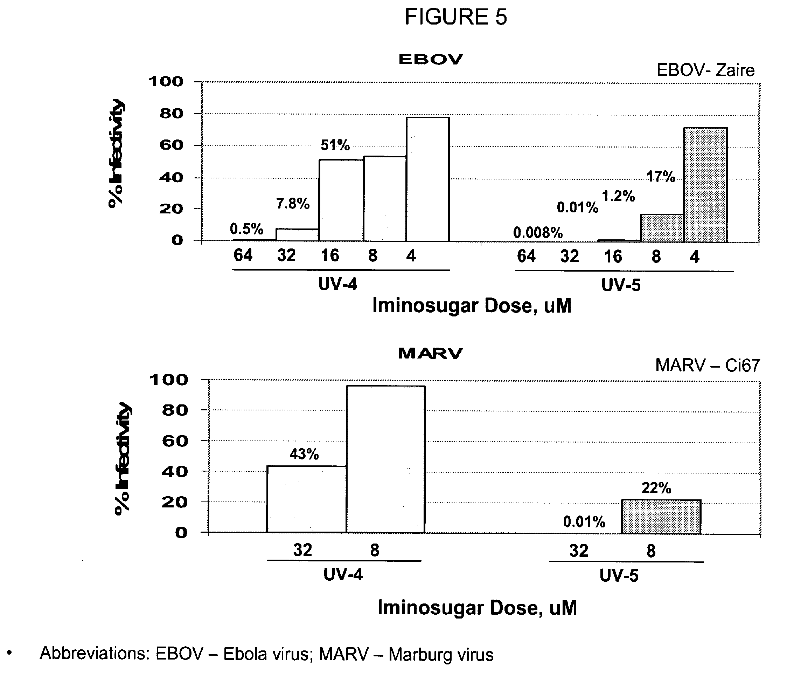

Figure 5 provides data for inhibition of infectivity of Ebola Zaire and Marburg viruses for N9-DNJ (UV-4) and NAP-DNJ (UV-5).

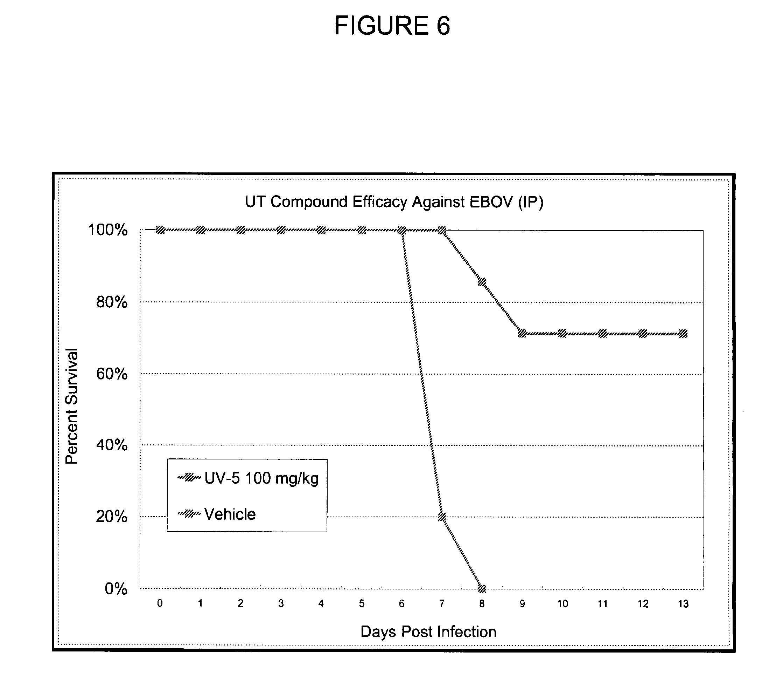

Figure 6 presents effects of 10 day administration of UV-5 on survival of mice infected with Ebola virus.

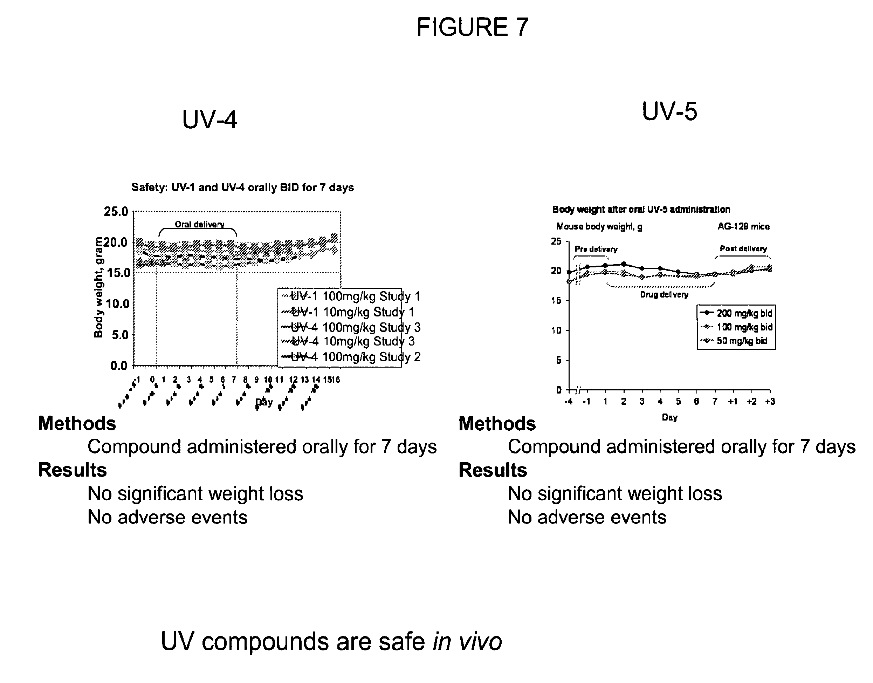

Figure 7 presents *in vivo* safety data for UV-4 and UV-5.

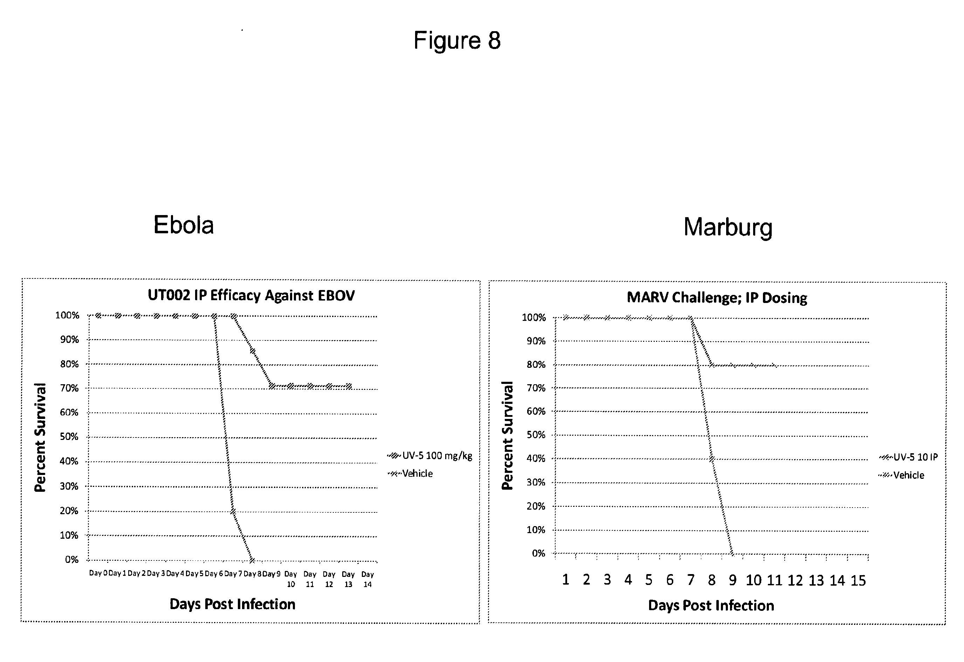

Figure 8 presents survival data for mice challenged with Ebola Zaire virus (left) and Marburg virus (right) after administering UV-5.

### DETAILED DESCRIPTION

### Related Documents

The following patent documents, may be useful for understanding the present disclosure:
1) US patent no. 6,545,021;
2) US patent no. 6,809,803;
3) US patent no. 6,689,759;
4) US patent no. 6,465,487;
5) US patent no. 5,622,972;
6) US patent application no. 12/656,992 filed February 22, 2010;
7) US patent application no. 12/656,993 filed February 22, 2010;
8) US patent application no. 12/813,882 filed June 11, 2010;
9) US patent provisional application no. 61/282,507 filed February 22, 2010;
10) US patent provisional application no. 61/272,252 filed September 4, 2009;
11) US provisional application no. 61/272,253 filed September 4, 2009;
12) US provisional application no. 61/272,254 filed September 4, 2009;
13) US provisional application no. 61/282,508 filed February 22,2010;
14) US provisional application no. 61/353,935 filed June 11, 2010.

### Definition of terms

Unless otherwise specified, "a" or "an" means "one or more."

As used herein, the term "viral infection" describes a diseased state, in which a virus invades a healthy cell, uses the cell's reproductive machinery to multiply or replicate and ultimately lyse the cell resulting in cell death, release of viral particles and the infection of other cells by the newly produced progeny viruses. Latent infection by certain viruses is also a possible result of viral infection.

As used herein, the term "treating or preventing viral infection" means to inhibit the replication of the particular virus, to inhibit viral transmission, or to prevent the virus from establishing itself in its host, and to ameliorate or alleviate the symptoms of the disease caused by the viral infection. The treatment is considered therapeutic if there is a reduction in viral load, decrease in mortality and/or morbidity.

IC50 or IC90 (inhibitory concentration 50 or 90) is a concentration of a therapeutic agent, such as an iminosugar, used to achieve 50% or 90% reduction of viral load, respectively.

### Disclosure

The present inventors discovered that certain iminosugars, such as deoxynojirimycin derivatives, may be effective against viruses belonging to the Filoviridae family, which are also known as filoviruses.

In particular, the deoxynojirimycin derivatives may be useful for treating or preventing a disease or condition caused by a virus belonging to the Filoviridae family.

The Filoviridae family includes the Ebolavirus genus and the Marburgvirus genus. The Ebolavirus genus includes Zaire virus, Bundibugyo Ebola virus, Ivory Coast Ebola virus, Reston Ebola virus and Sudan Ebola virus, while the Marburgvirus genus includes Lake Victoria Marburg virus. Diseases that are caused by filoviruses include Ebola hemorrhagic fever and Marburg hemorrhagic fever.

In many embodiments, the iminosugar may be N-substituted deoxynojirimycin. In some embodiments, such N-substituted deoxynojirimycin may be a compound of the following formula: 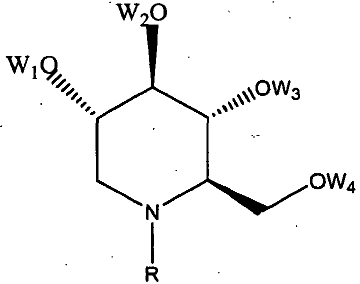 where W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

In some embodiments, R may be selected from substituted or unsubstituted alkyl groups, substituted or unsubstituted cycloalkyl groups, substituted or unsubstituted aryl groups, or substituted or unsubstituted oxaalkyl groups.

In some embodiments, R may be substituted or unsubstituted alkyl groups and/or substituted or unsubstituted oxaalkyl groups comprise from 1 to 16 carbon atoms, from 4 to 12 carbon atoms or from 8 to 10 carbon atoms. The term "oxaalkyl" refers to an alkyl derivative, which may contain from 1 to 5 or from 1 to 3 or from 1 to 2 oxygen atoms. The term "oxaalkyl" includes hydroxyterminated and methoxyterminated alkyl derivatives.

In some embodiments, R may be selected from, but is not limited to -(CH₂)₆OCH₃, -(CH₂)₆OCH₂CH₃, -(CH₂)₆O(CH₂)₂CH₃, -(CH₂)₆O(CH₂)₃CH₃, -(CH₂)₂O(CH₂)₅CH₃, -(CH₂)₂O(CH₂)₆CH₃;-(CH₂)₂O(CH₂)₇CH₃; -(CH₂)₉-OH; -(CH₂)₉OCH₃.

In some embodiments, R may be branched or unbranched, substituted or unsubstituted alkyl group, which may contain up 20 carbon atoms. In some embodiments, the alkyl group may be C2-C12 or C3-C7 alkyl group.

In certain embodiments, the alkyl group may be a long chain alkyl group, which may be C6-C20 alkyl group; C8-C16 alkyl group; or C8-C10 alkyl group. In some embodiments, R may be a long chain oxaalkyl group, i.e., a long chain alkyl group, which may contain from 1 to 5 or from 1 to 3 or from 1 to 2 oxygen atoms.

In some embodiments, R may have the following formula 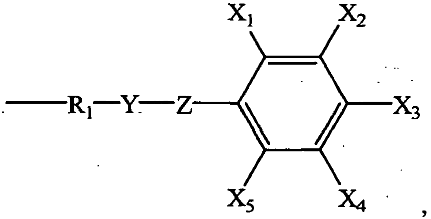 where R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl. In some embodiments, Z is NH and R₁-Y is a substituted or unsubstituted alkyl group, such as C2-C20 alkyl group or C4-C12 alkyl group or C4-C10 alkyl group.

In some embodiments, X₁ is NO₂ and X₃ is N₃. In some embodiments, each of X₂, X₄ and X₅ is hydrogen.

In some embodiments, the iminosugar may be a DNJ derivative disclosed in U.S. Patent application publication no. 2007/0275998.

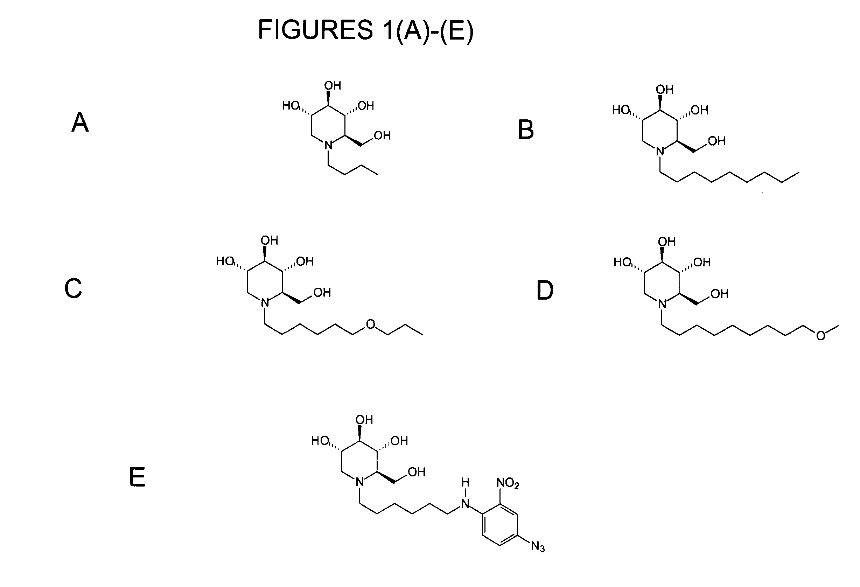

In some embodiments, the iminosugar may be one of the compounds presented in Figure 1. Methods of synthesizing deoxynojirimycin derivatives are disclosed, for example, in U.S.

Patent nos. 5,622,972, 5,200,523, 5,043,273, 4,994,572, 4,246,345, 4,266,025, 4,405,714, and 4,806,650 and U.S. Patent application publication no. 2007/0275998.

In some embodiments, the iminosugar may be in a form of a salt derived from an inorganic or organic acid. Pharmaceutically acceptable salts and methods for preparing salt forms are disclosed, for example, in Berge et al. (J. Pharm. Sci. 66:1-18, 1977). Examples of appropriate salts include but are not limited to the following salts: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate, and undecanoate.

In some embodiments, the iminosugar may also used in a form of a prodrug. Prodrug of DNJ derivatives, such as the 6-phosphorylated DNJ derivatives, are disclosed in U.S. Patents nos. 5,043,273 and 5,103,008.

In some embodiments, the iminosugar may be used as a part of a composition, which further comprises a pharmaceutically acceptable carrier and/ or a component useful for delivering the composition to an animal. Numerous pharmaceutically acceptable carriers useful for delivering the compositions to a human and components useful for delivering the composition to other animals such as cattle are known in the art. Addition of such carriers and components to the composition of the invention is well within the level of ordinary skill in the art.

In some embodiments, the pharmaceutical composition may consist essentially of *N-*substituted deoxynojirimycin, which may mean that the *N*-substituted deoxynojirimycin is the only active ingredient in the composition.

Yet in some embodiments, *N*-substituted deoxynojirimycin may be administered with one or more additional antiviral compounds.

In some embodiments, the treatment or prevention of the disease or condition caused by a virus belonging to the Filoviridae family may be performed without administering N-(phosphonoacetyl)-L-aspartic acid to the subject, to whom the iminosugar is being administered. N-(phosphonoacetyl)-L-aspartic acid is disclosed, for example, in U.S. patent no. 5,491,135.

In some embodiments, the iminosugar may be used in a liposome composition, such as those disclosed in US publications nos. 2008/0138351 and 2009/0252785 as well as in US application No. 12/732630 filed March 26, 2010.

The iminosugar, such as a DNJ derivative, may be administered to a cell or an animal affected by a virus. The iminosugar may inhibit morphogenesis of the virus, or it may treat the individual. The treatment may reduce, abate, or diminish the virus infection in the animal.

Animals that may be infected with a filovirus include primates, such as monkeys and humans. The amount of iminosugar administered to an animal or to an animal cell to the methods of the present disclosure may be an amount effective to inhibit the morphogenesis of a filovirus. The term "inhibit" as used herein may refer to the detectable reduction and/or elimination of a biological activity exhibited in the absence of the iminosugar. The term "effective amount" may refer to that amount of the iminosugar necessary to achieve the indicated effect. The term "treatment" as used herein may refer to reducing or alleviating symptoms in a subject, preventing symptoms from worsening or progressing, inhibition or elimination of the causative agent, or prevention of the infection or disorder related to the filovirus in a subject who is free therefrom.

Thus, for example, treatment of the disease caused by a virus may include destruction of the infecting agent, inhibition of or interference with its growth or maturation, and neutralization of its pathological effects. The amount of the iminosugar which may be administered to the cell or animal is preferably an amount that does not induce toxic effects which outweigh the advantages which accompany its administration.

Actual dosage levels of active ingredients in the pharmaceutical compositions may vary so as to administer an amount of the active compound(s) that is effective to achieve the desired therapeutic response for a particular patient.

The selected dose level may depend on the activity of the iminosugar, the route of administration, the severity of the condition being treated, and the condition and prior medical history of the patient being treated. However, it is within the skill of the art to start doses of the compound(s) at levels lower than required to achieve the desired therapeutic effect and to gradually increase the dosage until the desired effect is achieved. If desired, the effective daily dose may be divided into multiple doses for purposes of administration, for example, two to four doses per day. It will be understood, however, that the specific dose level for any particular patient can depend on a variety of factors, including the body weight, general health, diet, time and route of administration and combination with other therapeutic agents and the severity of the condition or disease being treated. The adult human daily dosage may range from between about one microgram to about one gram, or from between about 10 mg and 100 mg, of the iminosugar per 10 kilogram body weight. In some embodiments, a total daily dose may be from 0.1 mg/kg body weight to 100 mg/kg body weight or from 1 mg/kg body weight to 60 mg/kg body weight or from 2 mg/kg body weight to 50 mg/kg body weight or from 3 mg/kg body weight to 30 mg/kg body weight. The daily dose may be administered over one or more administering events over day. For example, in some embodiments, the daily dose may be distributed over two (BID) administering events per day, three administering events per day (TID) or four administering events (QID). In certain embodiments, a single administering event dose ranging from 1 mg/kg body weight to 10 mg/kg body weight may be administered BID or TID to a human making a total daily dose from 2 mg/kg body weight to 20 mg/kg body weight or from 3 mg/kg body weight to 30 mg/kg body weight. Of course, the amount of the iminosugar which should be administered to a cell or animal may depend upon numerous factors well understood by one of skill in the art, such as the molecular weight of the iminosugar and the route of administration. Pharmaceutical compositions that are useful in the methods of the present disclosure may be administered systemically in oral solid formulations, ophthalmic, suppository, aerosol, topical or other similar formulations. For example, it may be in the physical form of a powder, tablet, capsule, lozenge, gel, solution, suspension, syrup, or the like. In addition to the iminosugar, such pharmaceutical compositions may contain pharmaceutically-acceptable carriers and other ingredients known to enhance and facilitate drug administration. Other possible formulations, such as nanoparticles, liposomes, resealed erythrocytes, and immunologically based systems may also be used to administer the iminosugar. Such pharmaceutical compositions may be administered by a number of routes. The term "parenteral" used herein includes subcutaneous, intravenous, intraarterial, intrathecal, and injection and infusion techniques, without limitation. By way of example, the pharmaceutical compositions may be administered orally, topically, parenterally, systemically, or by a pulmonary route.

These compositions may be administered a in a single dose or in multiple doses which are administered at different times. Because the inhibitory effect of the composition upon a filovirus may persist, the dosing regimen may be adjusted such that virus propagation is retarded while the host cell is minimally effected. By way of example, an animal may be administered a dose of the composition of the invention once per week, whereby virus propagation is retarded for the entire week, while host cell functions are inhibited only for a short period once per week.

Embodiments described herein are further illustrated by, though in no way limited to, the following working examples.

### Working Examples

### 1. Synthesis of N-Nonyl DNJ

**Table 1. Materials for NN-DNJ synthesis**

| Name | Amount |
|---|---|
| DNJ | 500 mg |
| Nonanal | 530 mg |
| Ethanol | 100 mL |
| AcOH | 0.5 mL |
| Pd/C | 500 mg |

Procedure: A 50-mL, one-necked, round-bottom flask equipped with a magnetic stirrer was charged with DNJ (500 mg), ethanol (100 mL), nonanal (530 mg), and acetic acid (0.5 mL) at room temperature. The reaction mixture was heated to 40-45 °C and stirred for 30-40 minutes under nitrogen. The reaction mixture was cooled to ambient temperature and Pd/C was added. The reaction flask was evacuated and replaced by hydrogen gas in a balloon. This process was repeated three times. Finally, the reaction mixture was stirred at ambient temperature overnight. The progress of reaction was monitored by TLC (Note 1). The reaction mixture was filtered through a pad of Celite and washed with ethanol. The filtrate was concentrated *in vacuo* to get the crude product. The crude product was purified by column chromatography (230-400 mesh silica gel). A solvent gradient of methanol in dichloromethane (10-25%) was used to elute the product from the column. All fractions containing the desired product were combined, and concentrated *in vacuo* to give the pure product (420mg). Completion of the reaction was monitored by thin layer chromatography (TLC) using a thin layer silica gel plate; eluent; methanol: dichloromethane = 1:2

### 2. Synthesis of N-7-Oxadecyl DNJ

### 2a. Synthesis of 6-propyloxy-1-hexanol

**Table 2. Materials for synthesis of 6-propyloxy-1-hexanol**

| Name | Amount |
|---|---|
| 1,6-hexanediol | 6.00 g |
| 1-Iodopropane | 8.63 g |
| Potassium tert-butoxide | 5.413 mg |
| THF | 140 mL |

Procedure: a 500-mL, one-necked, round-bottom flask equipped with a magnetic stirrer was charged with 1,6-hexanediol (6.00 g), potassium tert-butoxide (5.413 g) at room temperature. The reaction mixture was stirred for one hour, and then 1-iodopropane (8.63 g) was added. The reaction mixture was heated to 70-80 °C and stirred overnight. The progress of reaction was monitored by TLC (Note 1). After completion of the reaction, water was added to the reaction mixture, and extracted with ethyl acetate (2 x 100 mL). The combined organic layers were concentrated *in vacuo* to get the crude product. The crude product was dissolved in dichloromethane and washed with water, and then brine, dried over sodium sulfate. The organic layer was concentrated *in vacuo* to get the crude product. The crude product was purified by column chromatography using 230-400 mesh silica gel. A solvent gradient of ethyl acetate in hexanes (10-45%) was used to elute the product from the column. All fractions containing the desired pure product were combined and concentrated *in vacuo* to give pure 6-propyloxy-1-hexanol (lot D-1029-048, 1.9 g, 25%) Completion of the reaction was monitored by thin layer chromatography (TLC); (eluent: 60% ethyl acetate in hexanes).

### 2b. Preparation of 6-propyloxy-1-hexanal

**Table 3. Materials for preparation of 6-propyloxy-1-hexanal**

| Name | Amount |
|---|---|
| 6-Propyloxy-1-hexanol | 1.00 g |
| PDC | 4.70 g |
| Celite | 1.00 g |
| NaOAc | 100 mg |
| CH₂Cl₂ | 10 mL |

Procedure: a 50-mL, one-necked, round-bottom flask equipped with a magnetic stirrer was charged with 6-propyloxy-1-hexanol (1.0 g), PDC (4.7 g), dichloromethane (10 mL), Celite (1.0 g), and sodium acetate (100 mg). The reaction mixture was stirred at room temperature under nitrogen for 5 minutes. PDC (4.70 g) was added to the reaction mixture, and stirred overnight. The progress of reaction was monitored by TLC (Note 1). After completion of the reaction, the reaction mixture was directly loaded on the column (230-400 mesh silica gel). A solvent gradient of dichloromethane in ethyl acetate (10-20%) was used to elute the product from the column. All fractions containing the desired pure product were combined and concentrated *in vacuo* to give pure 6-propyloxy-1-hexanal (lot D-1029-050, 710 mg, 71 %). Completion of the reaction was monitored by thin layer chromatography (TLC); (eluent: 60% ethyl acetate in hexanes).

### 2c Synthesis of N-7-Oxadecyl-DNJ

**Table 4. Materials for Synthesis of N-7-Oxadecyl-DNJ**

| Name | Amount |
|---|---|
| DNJ | 500 mg |
| 6-Propyloxy-1-hexanal | 585 mg |
| Pd/C | 125 mg |
| Ethanol | 15 mL |
| Acetic acid | mL |

Procedure: a 50-mL, one-necked, round-bottom flask equipped with a magnetic stirrer was charged with DNJ (500 mg), ethanol (15 mL), 6-propyloxy-1-hexanal (585 mg), and acetic acid (0.1mL) t room temperature. The reaction mixture was heated to 40-45 °C and stirred for 30-40 minutes under nitrogen. The reaction mixture was cooled to ambient temperature and Pd/C was added. The reaction flask was evacuated and replaced by hydrogen gas in a balloon. This process was repeated three times. Finally, the reaction mixture was stirred at ambient temperature overnight. The progress of reaction was monitored by TLC (Note 1). The reaction mixture was filtered through a pad of Celite and washed with ethanol. The filtrate was concentrated *in vacuo* to get the crude product. The crude product was purified by column chromatography (230-400 mesh silica gel). A solvent gradient of methanol in dichloromethane (10-40%) was used to elute the product from the column. All fractions containing the desired product were combined, and concentrated *in vacuo* to give the pure product. (Lot: D-1029-052 (840 mg). Completion of the reaction was monitored by thin layer chromatography (TLC); (eluent: 50% methanol in dichloromethane).

### 3. Synthesis of N-(9-methoxy)-nonyl DNJ

### 3a Preparation of 9-methoxy-1-nonanol

**Table 5. Materials for preparation of 9-methoxy-1-nonanol**

| Name | Amount |
|---|---|
| 1,9-nonanediol | 10.0 g |
| Dimethyl sulfate | 41.39 g |
| Sodium hydroxide | 5.0g |
| DMSO | 100 mL |

Procedure: a 500-mL, one-necked, round-bottom flask equipped with a magnetic stirrer and stir bar was charged with 1,9-nonanediol (10.00 g, 62.3 mmol) in dimethyl sulfoxide (100 mL) and H₂O (100 mL). To this was added slowly a solution of sodium hydroxide (5.0 g, 125.0 mmol) in H₂O (10 mL) at room temperature. During addition of sodium hydroxide the reaction mixture generated heat and the temperature rose to ~40 °C. The mixture was stirred for one hour, and then dimethyl sulfate (16.52 g, 131 mmol) was added in four portions while maintaining the temperature of the reaction mixture at ~ 40°C. The reaction mixture was stirred at room temperature overnight. Progress of the reaction was monitored by TLC (Note 1). TLC monitoring indicated that the reaction was 25 % conversion. At this stage additional dimethyl sulfate (24.78g, 196.44 mmol) was added and the resulting mixture was stirred at room temperature for an additional 24 h. After completion of the reaction, sodium hydroxide (10% solution in water) was added to the reaction mixture to adjust the pH of the solution to 11-13. The mixture was stirred at room temperature for 2 h and extracted with dichloromethane (3 x 100 mL). The combined organic layers were washed with H₂O (200 mL), brine (150 mL), dried over anhydrous sodium sulfate (20 g), filtered and concentrated *in vacuo* to obtain a crude product (14 g). The crude product was purified by column chromatography using 250-400 mesh silica gel. A solvent gradient of ethyl acetate in hexanes (10-50%) was used to elute the product from the column. All fractions containing the desired pure product were combined and concentrated *in vacuo* to give pure 9-methoxy-1-nonanol (lot D-1027-155, 2.38 g, 21.9 %). Completion of the reaction was monitored by thin layer chromatography (TLC) using a thin layer silica gel plate; eluent: 60% ethyl acetate in hexanes.

### 3b Preparation of 9-methoxy-1-nonanal

**Table 6. Materials for preparation of 9-methoxy-1-nonanal**

| Name | Amount |
|---|---|
| 9-methoxy-1-nonanol | 1.0 g |
| PDC | 4.7 g |
| Molecular sieves, 3A | 1.0 g |
| NaOAc | 0.1g |
| CH₂Cl₂ | 10 mL |

Procedure: a 50-mL, one-necked, round-bottom flask equipped with a magnetic stirrer and stir bar was charged with 9-methoxy-nonanol (1.0 g, 5.9 mmol), dichloromethane (10 mL), molecular sieves (1.0 g, 3A), sodium acetate (0.1 g) at room temperature. The reaction mixture was stirred at room temperature under nitrogen for 5 minutes. The reaction mixture was charged with pyridinium dichromate (4.7 g, 12.5 mmol) and stirred overnight. The progress of reaction was monitored by TLC (Note 1). After completion of the reaction, the reaction mixture was filtered through a bed of silica gel (~15 g). The filtrate was evaporated in *vacuo* to obtain a crude compound. This was purified by column chromatography using silica gel column (250-400 mesh, 40 g). A solvent gradient of ethyl acetate in hexane (10-50%) was used to elute the product from the column. All fractions containing the desired pure product were combined and concentrated *in vacuo* to give pure 9-methoxy-nonanal (lot D-1027-156, 553 mg, 54.4%). Completion of the reaction was monitored by thin layer chromatography (TLC) using a thin layer silica gel plate; eluent: 60% ethyl acetate in hexanes.

### 3c Synthesis of N-(9-methoxy)-nonyl DNJ

**Table 7. Materials for synthesis of N-(9-methoxy)-nonyl DNJ**

| Name | Amount |
|---|---|
| DNJ | 300 mg |
| 9-methoxy-1-nonanal | 476 mg |
| Pd/C | 200 mg |
| Ethanol | 20 mL |

Procedure: a 50-mL, two-necked, round-bottom flask equipped with magnetic stirrer and a stir bar was charged with DNJ (300 mg, 1.84 mmol), ethanol (20 mL), 9-methoxy-1-nonanal (476 mg, 2.76 mmol) at room temperature. The reaction mixture was stirred for 5-10 minutes under nitrogen and Pd/C was added at room temperature. The reaction mixture was evacuated and was replaced by hydrogen gas using a balloon. This process was repeated three times and then reaction mixture was stirred under atmospheric hydrogen at room temperature. The progress of reaction was monitored by TLC (Note 1). The reaction mixture was filtered through a bed of Celite and was washed with ethanol (20 mL). The filtrate was concentrated *in vacuo* to get a crude product. The crude product was purified by column chromatography using 250-400 mesh silica gel (20 g). A solvent gradient of methanol in ethyl acetate (5-25%) was used to elute the product from the column. All fractions containing the desired pure product were combined, and concentrated *in vacuo* to give an off white solid. The solid was triturated in ethyl acetate (20 mL), filtered and dried in high vacuum to give a white solid [lot: D-1027-158 (165.3 mg, 28.1%). Completion of the reaction was monitored by thin layer chromatography (TLC) using a thin layer silica gel plate; eluent: 50% methanol in dichloromethane.

### 4. Inhibition of Ebola (Zaire) and Marburg viruses

Table 1 presents IC50 values for Ebola Zaire and Marburg viruses in µM. The table provides data for inhibition of infectivity of Ebola Zaire and Marburg viruses for NB-DNJ (UV-1), NN-DNJ (UV-2), N7-O-DNJ (UV-3), N9-DNJ (UV-4) and NAP-DNJ (UV-5).

| Compound | Ebola Zaire Virus | Marburg Ci67 |
|---|---|---|
| NB-DNJ | 32 | Not available |
| NN-DNJ | 12 | Not available |
| N7-O-DNJ | 20 | 50 |
| N9-DNJ | 16 | 32 |
| NAP-DNJ | 6 | 6 |

Procedure. The compounds were screened for inhibition of generation of infectious virus was conducted on the UV compounds at concentrations from 6 µM up to 250 µM. The filovirus Ebola-Zaire and Marburg-Ci67 strains were evaluated for virus inhibition. Vero cells (African green monkey kidney epithelial cell line) obtained from American Type Culture Collection (ATCC, Manassas, Virginia). Cells were cultured in 1x modified Eagle medium (MEM, Gibco), supplemented with 2% fetal bovine serum, 2 mM L-glutamine, 100 U/ml penicillin, 100 µg/ml streptomycin in cell culture treated 24-well flat bottom plates at 37°C in a 5% CO2 incubator for 24 hr prior to assay. Cells (1x10⁶ cells per well) were pretreated with compounds in a final concentration of 1 % DMSO for 1 hr followed by discarding the culture medium and addition of virus inoculums at an MOI of 0.1 in EMEM with 2% FBS. After 1 hr incubation the virus inoculums were removed and fresh media with compounds at correct dilutions were added. Three days later virus containing supernatants were collected and 10 fold dilutions of virus-containing supernatants was done in a virus plaque assay with Vero cells plated on 6 well- virus plaque assay plates. The plaque assay data were collected on day 8 for the Ebola and Marburg plates. IC 50 was determined as concentration of compound resulting in 50% virus inhibition.

Figure 5 provides data for inhibition of infectivity of Ebola Zaire and Marburg viruses for N9-DNJ (UV-4) and NAP-DNJ (UV-5).

Procedure. The virus yield assay were performed by standard plaque assay on supernatant samples generated from virus-infected cells incubated with iminosugars at concentrations from 4 µM up to 64 µM. The filovirus Ebola-Zaire and Marburg-Ci67 strains were evaluated for virus inhibition. 24-well cell culture plates were seeded with Vero cells (ATCC, Mannassas, VA; ATCC number CCL-81) in 1mL Ix modified Eagle medium (MEM, Gibco), supplemented with 10% heat-inactivated fetal bovine serum, 2 mM L-glutamine, 100 U/ml penicillin, 100 µg/ml streptomycin and incubated at 37°C for 24 hours or until ~80% confluency. Medium were replaced with medium supplemented with 2% fetal bovine serum and the cells were pretreated with compounds in a final concentration of 1% DMSO for 1hr followed by discarding the culture medium and addition of virus inoculums at an MOI of 0.1 in triplicate and incubated for 1hr at 37°C, 5% CO₂. After 1 hr incubation the virus inoculums were removed and fresh media with compounds at correct dilutions were added. Three days are required for the EBOV and MARV virus infection. Upon completion of infection, supernatant were collected for titering. To titer, 6-well plates with 80% confluent Vero cells in growth medium were used. Viral supernatant were diluted from 10⁻³ to 10⁻⁸ and added (100 uL) to the cells and incubated at 37°C for 1 hour with shaking every 5-10 minutes. Viral infection medium (100 uL) were aspirated and replace with 1 mL pre-warmed 2% low-melt agarose mixed 1:1 with 2X MEM (5% fetal calf serum) and incubated at 37°C, 5% CO₂ for 8 days followed by plaque visualization by neutral red staining.

### Ebola In Vivo Study

UV-5 was administered as a free drug dissolved in water. The compound was given at 100mg/kg and 10mg/kg by the intraparenteral route (IP) twice daily. Balb/c mice received the compound for 10 days. Mice were infected with Ebola virus (strain Zaire) with ~5 LD50 30 minutes following the first iminosugar dose. Animals were monitored for 15 days. Animals were weighed once per day, and given health scores 2X per day. Animals displaying severe illness (as determined by 30% weight loss, extreme lethargy, ruffled coat, or paralysis) were euthanized.

Figure 6 presents data for the effects of 10 day administration of UV-5 on survival of mice infected with Ebola virus. Animals receiving 100 mg/kg and 10 mg/kg BID showed a 71% survival rate, versus no survival in control animals.

Conclusion: these results demonstrate that UV-5 can be used as an antiviral drug to treat Ebola.

### Iminosugar Safety Study

Methods and Discussion: BALB/c and C57/B1/6 mice were given oral suspensions of UV-1, UV-4, UV-5, twice a day for seven days, in 100ul per mouse at 100 and 10 mg/kg (2mg and 0.2 mg/mouse, respectively) 8 hours apart for 7 days, and then monitored for weight loss and general health. After seven days of treatment, the mice did not show any significant signs of weight loss compared to the "vehicle only" control. The results of these experiments are in Figure 7.

When the BALB/c mice were treated with UV-5 at the highest concentration, they displayed signs of diarrhea, red urine, and a ruffled appearance although they did not show signs of weight loss. The C57/B1/6 mice displayed these same symptoms but without the ruffled look. These symptoms promptly ceased when treatment was done, and by day 11 (day 4 post compound treatment) the BALB/c mice in these groups looked very healthy.

Conclusions: These compounds have shown to be relatively non-toxic in this mouse model and these concentrations of compound are deemed safe.

### Filoviridae In Vivo Data

The study assessed the efficacy of the iminosugar compound UV-5 in promoting survival of mice challenged with Ebola and Marburg viruses. C57B1/6 mice were used in the Ebola experiments, while Balb/c mice were used in the Marburg virus experiments. UV-5 compound was previously tested in both in vitro (CC50 of 125-250uM) and in vivo (no weight loss or adverse effects observed in multiple mouse studies) and shown it possesses low toxicity. In this study, UV-5 compound was administered to the mice as a free drug dissolved in PBS. The compound was be given by the intraperitoneal (IP) route (2x per day IP) for a total number of 10 days after the start of the compound dosing. Study mice were infected IP with Ebola Zaire or Marburg Ravn with 1000 pfu/mouse 1h before the first UV-5 dose.

Animals were monitored for 15 days. Animals were weighed once per day, and given health scores 2X per day. Animals displaying severe illness (as determined by 30% weight loss, extreme lethargy, ruffled coat, or paralysis) were euthanized.

Figure 8 shows survival data (Y-axis, percent of mice in a study group survived, X-axis, the number of days post infection) for mice infected with Ebola Zaire or Marburg Ravn viruses. Each of the groups in the study, i.e. i) a control group (treated with water only) infected with the Ebola virus, ii) a control group (treated with water only) infected with the Marburg virus; iii) a treated group (treated with 100 mg/kg of UV 5, BID) infected with the Ebola virus; iv) a treated group (treated with 10 mg/kg of UV 5, BID) infected with the Marburg virus, contained 10 mice at the beginning of the study.

Survival of ≥60% is statistically significant. UV-5 provided significant protection against Ebola virus at dosing of 100 mg/kg IP, BID. UV-5 provided protection against Marburg virus at dosing of 10 mg/kg IP, BID.

## Claims

1. A compound having the formula, 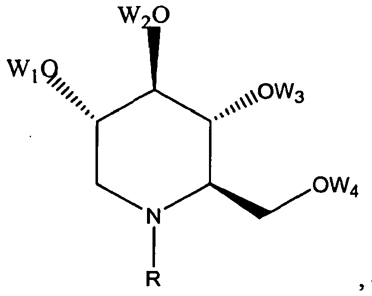 or a pharmaceutically acceptable salt thereof, for use in a method of treating or preventing in a subject a disease or condition caused by a virus belonging to the Filoviridae family,
wherein R is (a) 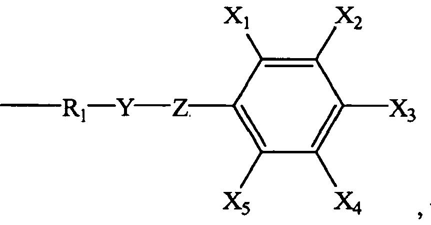 wherein
R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl; or
(b) either selected from substituted or unsubstituted alkyl groups, substituted or unsubstituted cycloalkyl groups, substituted or unsubstituted aryl groups, or substituted or unsubstituted oxaalkyl groups; and
wherein W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

2. The compound according to claim 1, for use according to claim 1, wherein each of W₁, W₂, W₃ and W₄ is hydrogen.

3. The compound according to claim 1 for use according to claim 1, wherein R is

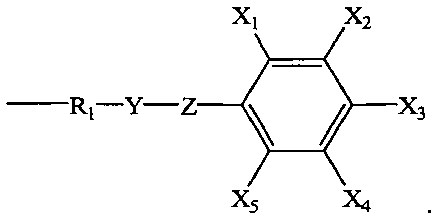

4. The compound according to claim 3 for use according to claim 3, wherein X₁ is NO₂ and X₃ is N₃.

5. The compound according to claim 3 for use according to claim 3, wherein each of X₂, X₄ and X₅ is hydrogen.

6. The compound according to claim 3 for use according to claim 3, which is *N-*(*N*-{4'-azido-2'-nitrophenyl}-6-aminohexyl)deoxynojirimycin or a pharmaceutically acceptable salt thereof.

7. The compound according to claim 1 for use according to claim 1, wherein R is selected from substituted or unsubstituted alkyl groups, substituted or unsubstituted cycloalkyl groups, substituted or unsubstituted aryl groups, or substituted or unsubstituted oxaalkyl groups.

8. The compound according to claim 7 for use according to claim 7, wherein R is C2-C 12 alkyl group.

9. The compound according to claims 1-2 for use according to any one of claims 1-2, wherein said compound is (i) B-butyl deoxynojirimycin or a pharmaceutically acceptable salt thereof or (ii) B-nonyl deoxynojirimycin or a pharmaceutically acceptable salt thereof.

10. The compound according to claim 7 for use according to claim 7, wherein R is an oxaalkyl group, preferably C2-C 16 oxaalkyl group that contains from 1 to 3 oxygen atoms, more preferably C6-C12 oxaalkyl group that contains from 1 to 2 oxygen atoms.

11. The compound according to claims 1-2 for use according to any one of claims 1-2, wherein said compound is (iii) N-(7-oxadecyl)deoxynojirimycin or a pharmaceutically acceptable salt thereof; or (iv) N-(9-Methoxynonyl)deoxynojirimycin or a pharmaceutically acceptable salt thereof.

12. The compound according to claims 1-11 for use according to any one of claims 1-11, wherein the virus is a Marburg virus.

13. The compound according to claims 1-11 for use according to any one of claims 1-11, wherein the virus belongs to the Ebola virus family, preferably the virus is a Zaire virus.

14. The compound according to claims 1-13 for use according to any one of claims 1-13, wherein the subject is a mammal, preferably a human being.

## Patentansprüche

1. Verbindung mit der Formel 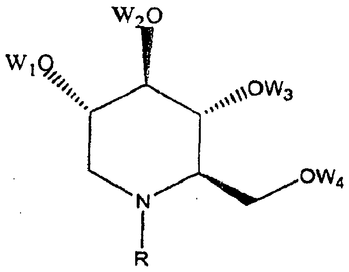 oder pharmazeutisch annehmbares Salz davon zur Verwendung in einem Verfahren zur Behandlung oder Vorbeugung einer Krankheit oder eines Zustands, die/der durch ein zu der Filoviridae-Familie gehörendes Virus verursacht wird, in einem Patienten, wobei R
(a) 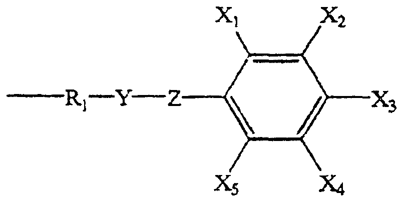 ist, wobei
R₁ eine substituierte oder unsubstituierte Alkylgruppe ist;
X₁₋₅ unabhängig aus H, NO₂, N₃ oder NH₂ ausgewählt werden;
Y nicht vorhanden ist oder eine substituierte oder unsubstituierte C₁-Alkylgruppe, die von Carbonyl verschieden ist, ist; und
Z aus einer Bindung oder NH ausgewählt wird; vorausgesetzt, dass wenn Z eine Bindung ist, Y nicht vorhanden ist, und vorausgesetzt, dass wenn Z NH ist, Y eine substituierte oder unsubstituierte C₁-Alkylgruppe, die von Carbonyl verschieden ist,
ist; oder
(b) aus entweder substituierten oder unsubstituierten Alkylgruppen, substituierten oder unsubstituierten Cycloalkylgruppen, substituierten oder unsubstituierten Arylgruppen oder substituierten oder unsubstituierten Oxaalkylgruppen ausgewählt wird; und
wobei W₁₋₄ unabhängig aus Wasserstoff, substituierten oder unsubstituierten Alkylgruppen, substituierten oder unsubstituierten Haloalkylgruppen, substituierten oder unsubstituierten Alkanoylgruppen, substituierten oder unsubstituierten Aroylgruppen oder substituierten oder unsubstituierten Haloalkanoylgruppen ausgewählt werden.

2. Verbindung gemäß Anspruch 1 zur Verwendung gemäß Anspruch 1, wobei jedes von W₁, W₂, W₃ und W₄ Wasserstoff ist.

3. Verbindung gemäß Anspruch 1 zur Verwendung gemäß Anspruch 1, wobei R 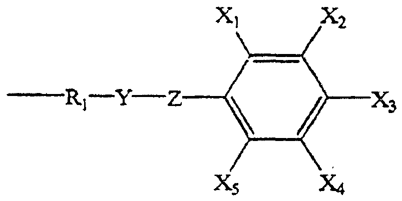 ist.

4. Verbindung gemäß Anspruch 3 zur Verwendung gemäß Anspruch 3, wobei X₁ NO₂ ist und X₃ N₃ ist.

5. Verbindung gemäß Anspruch 3 zur Verwendung gemäß Anspruch 3, wobei jedes von X₂, X₄ und X₅ Wasserstoff ist.

6. Verbindung gemäß Anspruch 3 zur Verwendung gemäß Anspruch 3, die N-(N-{4'-Azido-2'-nitrophenyl}-6-aminohexyl)deoxynojirimycin oder ein pharmazeutisch annehmbares Salz davon ist.

7. Verbindung gemäß Anspruch 1 zur Verwendung gemäß Anspruch 1, wobei R aus substituierten oder unsubstituierten Alkylgruppen, substituierten oder unsubstituierten Cycloalkylgruppen, substituierten oder unsubstituierten Arylgruppen oder substituierten oder unsubstituierten Oxaalkylgruppen ausgewählt wird.

8. Verbindung gemäß Anspruch 7 zur Verwendung gemäß Anspruch 7, wobei R eine C2-C12-Alkylgruppe ist.

9. Verbindung gemäß einem der Ansprüche 1 bis 2 zur Verwendung gemäß einem der Ansprüche 1 bis 2, wobei die Verbindung (i) B-Butyldeoxynojirimycin oder ein pharmazeutisch annehmbares Salz davon oder (ii) B-Nonyldeoxynojirimycin oder ein pharmazeutisch annehmbares Salz davon ist.

10. Verbindung gemäß Anspruch 7 zur Verwendung gemäß Anspruch 7, wobei R eine Oxaalkylgruppe, vorzugsweise eine C2-C16-Oxaalkylgruppe, die 1 bis 3 Sauerstoffatome enthält, stärker bevorzugt eine C6-C12-Oxaalkylgruppe, die 1 bis 2 Sauerstoffatome enthält, ist.

11. Verbindung gemäß den Ansprüchen 1 bis 2 zur Verwendung gemäß gemäß den Ansprüchen 1 bis 2, wobei die Verbindung (iii) N-(7-Oxadecyl)deoxynojirimycin oder ein pharmazeutisch annehmbares Salz davon oder (iv) N-(9-Methoxynonyl)deoxynojirimycin oder ein pharmazeutisch annehmbares Salz davon ist.

12. Verbindung gemäß den Ansprüchen 1 bis 11 zur Verwendung gemäß den Ansprüchen 1 bis 11, wobei das Virus ein Marburg-Virus ist.

13. Verbindung gemäß den Ansprüchen 1 bis 11 zur Verwendung gemäß den Ansprüchen 1 bis 11, wobei das Virus zu der Ebola-Virus-Familie gehört und das Virus vorzugsweise ein Zaire-Virus ist.

14. Verbindung gemäß den Ansprüchen 1 bis 13 zur Verwendung gemäß den Ansprüchen 1 bis 13, wobei der Patient ein Säugetier, vorzugsweise ein Mensch ist.

## Revendications

1. Composé répondant à la formule, 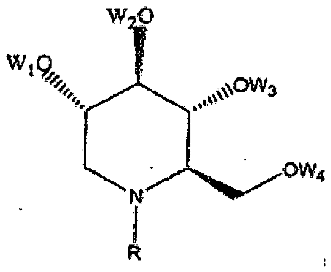 , ou un sel pharmaceutiquement acceptable de celui-ci, pour une utilisation dans une méthode de traitement ou de prévention chez un sujet d'une maladie ou d'une affection provoquée par un virus appartenant à la famille des Filoviridae,
dans laquelle R est (a) 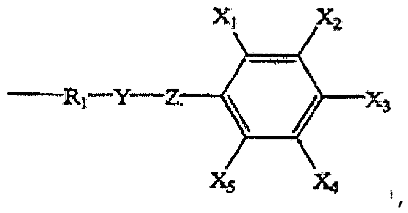 dans laquelle
R₁ est un groupe alkyle substitué ou non substitué ;
X₁₋₅ sont choisis indépendamment parmi H, NO₂, N₃, ou NH₂ ;
Y est absent ou est un groupe alkyle en C₁ substitué ou non substitué, autre que le carbonyle ; et
Z est choisi parmi une liaison ou NH ; à condition que lorsque Z est une liaison, Y soit absent, et à condition que lorsque Z est NH, Y soit un groupe alkyle en C₁ substitué ou non substitué, autre que le carbonyle ; ou
(b) choisi parmi les groupes alkyle substitués ou non substitués, les groupes cycloalkyle substitués ou non substitués, les groupes aryle substitués ou non substitués, ou les groupes oxaalkyle substitués ou non substitués ; et
dans laquelle W₁₋₄ sont choisis indépendamment parmi l'hydrogène, les groupes alkyle substitués ou non substitués, les groupes halogénoalkyle substitués ou non substitués, les groupes alcanoyle substitués ou non substitués, les groupes aroyle substitués ou non substitués, ou les groupes halogénoalcanoyle substitués ou non substitués.

2. Composé selon la revendication 1, pour une utilisation selon la revendication 1, dans lequel chacun de W₁, W₂, W₃ et W₄ est un atome d'hydrogène.

3. Composé selon la revendication 1 pour une utilisation selon la revendication 1, dans lequel R est

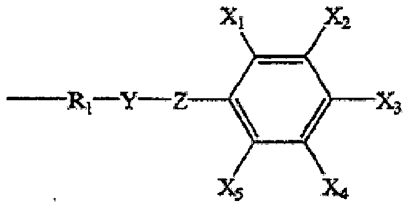

4. Composé selon la revendication 3 pour une utilisation selon la revendication 3, dans lequel X₁ est NO₂ et X₃ est N₃.

5. Composé selon la revendication 3 pour une utilisation selon la revendication 3, dans lequel chacun de X₂, X₄ et X₅ est un atome d'hydrogène.

6. Composé selon la revendication 3 pour une utilisation selon la revendication 3, qui est la *N*-(*N*-{4'-azido-2'-nitrophényl}-6-aminohexyl)désoxynojirimycine ou un sel pharmaceutiquement acceptable de celui-ci.

7. Composé selon la revendication 1 pour une utilisation selon la revendication 1, dans lequel R est choisi parmi les groupes alkyle substitués ou non substitués, les groupes cycloalkyle substitués ou non substitués, les groupes aryle substitués ou non substitués, ou les groupes oxaalkyles substitués ou non substitués.

8. Composé selon la revendication 7 pour une utilisation selon la revendication 7, dans lequel R est un groupe alkyle en C2-C12.

9. Composé selon les revendications 1 à 2 pour une utilisation selon l'une quelconque des revendications 1 à 2, dans lequel ledit composé est (i) la B-butyl désoxynojirimycine ou un sel pharmaceutiquement acceptable de celle-ci ou (ii) la B-nonyl désoxynojirimycine ou un sel pharmaceutiquement acceptable de celle-ci.

10. Composé selon la revendication 7 pour une utilisation selon la revendication 7, dans lequel R est un groupe oxaalkyle, de préférence un groupe oxaalkyle en C2-C16 qui contient de 1 à 3 atomes d'oxygène, plus préférablement un groupe oxaalkyle en C6-C12 qui contient de 1 à 2 atomes d'oxygène.

11. Composé selon les revendications 1 à 2 pour une utilisation selon l'une quelconque des revendications 1 à 2, dans lequel ledit composé est (iii) la N-(7-oxadécyl)désoxynojirimycine ou un sel pharmaceutiquement acceptable de celle-ci ; ou (iv) la N-(9-méthoxynonyl)désoxynojirimycine ou un sel pharmaceutiquement acceptable de celle-ci.

12. Composé selon les revendications 1 à 11 pour une utilisation selon l'une quelconque des revendications 1 à 11, dans lequel le virus est un virus Marburg.

13. Composé selon les revendications 1 à 11 pour une utilisation selon l'une quelconque des revendications 1 à 11, dans lequel le virus appartient à la famille des virus Ebola, de préférence le virus est un virus du Zaïre.

14. Composé selon les revendications 1 à 13 pour une utilisation selon l'une quelconque des revendications 1 à 13, dans lequel le sujet est un mammifère, de préférence un être humain.
